# EUROPEAN PATENT SPECIFICATION

(11) **EP 2 980 588 B1**
(45) Date of publication and mention of the grant of the patent: **18.10.2017**
(21) Application number: 14382299.7
(22) Date of filing: 31.07.2014
(51) Int. Cl.: G01N 33/569, G01N 33/543

(54) **Method and apparatus for detection of microorganisms**
Verfahren und Vorrichtung zum Nachweis von Mikroorganismen
Procédé et appareil de détection de micro-organismes

(43) Date of publication of application: 03.02.2016
(73) Proprietor: Betelgeux, S. L., 46701 Gandia Valencia (ES); Photek Limited, Sussex TN38 9NS (GB); Société 40-30, 38176 Seyssinet-Pariset (FR)
(72) Inventor: Orihuel Iranzo, Enrique, 46701 Gandia (Valencia) (ES); Lorenzo Carton, Fernando, 46701 Gandia (Valencia) (ES); Ingle, Martin, St. Leonards on Sea East Sussex TN38 9NS (GB); Sundararajan, Shobitha, St. Leonards on Sea East Sussex TN38 9NS (GB); Blanc, Séverine, 38176 Seyssinet-Pariset (FR); Hury, Stéphane, 38176 Seyssinet-Pariset (FR); Giao, Maria S., Southampton Hampshire SO17 1BJ (GB); Keevil, Charles W., Southampton Hampshire SO17 1BJ (GB); Gilmartin, Niamh, Dublin 9 (IE); O'Kennedy, Richard, Dublin 9 (IE); Belenguer Ballester, José, 46980 Paterna (Valencia) (ES); Porta Banderas, Sonia, 46980 Paterna (Valencia) (ES)
(74) Representative: Ungria López, Javier

(56) References cited:
- US-A1- 2003 134 434
- US-A1- 2009 176 255
- US-A1- 2009 232 852
- US-A1- 2013 011 862
- US-B2- 7 989 221
- HYUN-SOO KIM ET AL: "A rapid and sensitive immunoassay for detection of E. coli O157:H7 using multienzyme - Au nanoparticle complex", BIOCHIP JOURNAL, vol. 8, no. 1, 1 March 2014 (2014-03-01), pages 1-7, XP055165983, ISSN: 1976-0280, DOI: 10.1007/s13206-014-8101-7
- LAUBE TAMARA ET AL: "Phagomagnetic immunoassay for the rapid detection ofSalmonella", APPLIED MICROBIOLOGY AND BIOTECHNOLOGY, SPRINGER, DE, vol. 98, no. 4, 21 December 2013 (2013-12-21), pages 1795-1805, XP035328455, ISSN: 0175-7598, DOI: 10.1007/S00253-013-5434-4 [retrieved on 2013-12-21]
- M. FREITAS ET AL: "Iron oxide/gold core/shell nanomagnetic probes and CdS biolabels for amplified electrochemical immunosensing of Salmonella typhimurium", BIOSENSORS AND BIOELECTRONICS, vol. 51, 1 January 2014 (2014-01-01), pages 195-200, XP055165990, ISSN: 0956-5663, DOI: 10.1016/j.bios.2013.07.048
- WANG ZHOULI ET AL: "Preparation of immunomagnetic nanoparticles for the separation and enrichment ofAlicyclobacillusspp. in apple juice", FOOD RESEARCH INTERNATIONAL, vol. 54, no. 1, 1 November 2013 (2013-11-01), pages 302-310, XP028750333, ISSN: 0963-9969, DOI: 10.1016/J.FOODRES.2013.07.021

## Description

### TECHNICAL FIELD OF THE INVENTION

The present invention falls within the field of Health, especially Health in the food industry, in regard to the detection, control and elimination of pathogens and biological contaminants in environments of processing and storage of foods, specifically in surfaces such as hard surfaces. Nevertheless, the invention also falls within other many fields in which the detection of unwanted microorganisms on surfaces is useful, for instance pharmaceutics, hospitals or environmental control, among others.

### BACKGROUND OF THE INVENTION

### Importance of control of pathogens in food industries

In recent years, a series of negative incidents concerning food safety has heightened consumer's awareness of food commodities and their production. Apart from increasing concerns about food safety, often fuelled by mass media reports, changes in legislation created the opportunity for quality control laboratories and manufacturers of equipment for analytical assays to provide assurance along the entire food supply chain from the farm to retailer's shelf. Food safety control is a major economic activity with a volume of $2 billion and a growth rate of 12 %. The detection and identification of food-borne pathogens continues to rely on conventional culturing techniques. These are very elaborate, time-consuming and expensive. The existing food quality control laboratories use conventional techniques, providing a limited range of analyses. For that reason they are unable to cover the needs of the domestic market, thus the offer to demand ratio is extremely low. Consequently, there is a major requirement for simple, rapid, specific, sensitive and economically feasible methods.

The Codex Alimentarius Commission (CAC) defined food hygiene as "all conditions and measures necessary to ensure the safety and suitability of food at all stages of the food chain" In addition, the EU's General Food Hygiene Directive defined food hygiene as "all measures necessary to ensure the safety and wholesomeness of foodstuffs".

The preventive approach to the problem involves examining every stage of the process where contamination can occur in order to assure that the final product is safe. In this line, the analysis of the production and processing environments is one of the most effective ways to identify and prevent the presence of pathogenic microorganisms in foodstuffs, in particular of pathogenic microorganisms such as *Salmonella, Campylobacter, Escherichia coli* or *Listeria monocytogenes.* This is even more crucial if we bear in mind their ability to attach to surfaces and form biofilms.

### Current techniques

Conventional methods in microbiological analysis are used despite their long turnover times because of their high selectivity and sensitivity. Rapid methods, in particular biosensors have the potential to shorten the time span between sample uptake and results, but their future lies in reaching selectivity and sensitivity comparable to established methods at a fraction of the cost. Although not so critical, issues such as ease of use, low maintenance and continuous operation also need to be considered.

Established methods include the polymerase chain reaction (PCR), culture and colony counting methods as well as immunology-based methods are the most common tools used for pathogen detection. They involve DNA analysis, counting of bacteria and antigen-antibody interactions, respectively. In spite of disadvantages such as the time required for the analysis or the complexity of their use, they still represent a field where progress is possible. These methods are often combined together to yield more robust results. Recent developments in the field include combinations of PCR with mass spectrometry (MS), impedance/conductivity measurements, microcalorimetry, flow cytometry, nuclease/lysate tests, adenosine triphosphate (ATP) measurement, luminescence/fluorescence, molecular biological tests and immunoassays.

Generally, rapid methods aspire to replace (or, at least, complement) conventional assays such as ELISA tests and cell cultures. In the latter case, identification of a bacterial population requires several weeks, which can be reduced to a mere 5-48 hrs by using an appropriate advanced bioassay system. However, assay time is inversely correlated with the size of the population under investigation, with an average capacity of detecting 10²-10⁴ cells/mL within a time frame of 5-7 hrs.

Traditional methods currently account for approximately 65% of the tests performed worldwide in 2005 in the food microbiology market. Rapid methods accounted for the remaining 35%, or approximately 220 million tests. It is expected, however, that by 2010 traditional methods will represent only 52% of all tests, with a total growth rate of 11%, whereas the market share of rapid methods will grow three times faster, reaching 394.6 million tests in 2010.

Despite the efficiency of the above methods, their application commonly requires well-trained personnel and high capital investment for purchase of advanced scientific equipment. So far, they have been mostly applied purely for research purposes, providing useful data for scientific community and surveillance purposes. A number of instruments for the detection of food pathogens such as *Listeria monocytogenes* have been commercially available for some years, such as:
- A flow-through immunocapture system called Pathatrix® (MatrixMicroScience Ltd., Cambridgeshire, UK).
- An automated immunomagnetic capture system called BeadRetriever (Dynal Biotech Ltd., UK) has been used for the detection.

A summary of most prominent biosensor technologies already commercially applied in food microbiological safety in the USA is given in Table 1. It is obvious that immunosensors are the method of choice, although this may change as alternative technologies (e.g. impedance-based sensors), are becoming the favorites of many researchers and companies worldwide.

**Table 1. Patent and patent applications related to biosensor technologies for the detection of pathogens (L. Monocytogenes) in the USA**

| **Patent number** | **Title** |
|---|---|
| 5089386 | Test for Listeria |
| | Nucleic acid fragment capable of hybridizing to rRNA of *Listeria monocytogenes* and not to rRNA of *Bacillus subtilis.* |
| 20070259393 | Plating media for the presumptive identification of *Listeria* sp, *Listeria monocytogenes* and *Listeria ivanovii* |
| 5389513 | Method for detecting *Listeria monocytogenes* |
| | A DNA probe is disclosed which is capable of hybridizing to a portion of the genome of pathogenic *Listeria monocytogenes* but which does not hybridize to portions of the genomes of other Listeria. |
| 5139933 | Assay method for detecting Listeria |
| | An assay method is provided to quickly detect the presence of Listeria strains in samples, characterized by the use of antibodies to selectively capture the peptidoglycan and teichoic acid. |
| 20070254320 | Method and Kit for Detecting *Listeria* Spp. |
| | The subject invention is directed to a method and a kit for detecting *Listeria* spp. in food samples, biological sample (e.g., blood, saliva, tissue samples, cells samples, etc.). |
| 20060286559 | Nucleic acids for detection of Listeria |
| 5922538 | Genetic markers and methods for the detection of *Listeria monocytogenes* and Listeria spp |
| 20090203028 | Aptamers bind to Listeria surface proteins. |
| 5376528 | Probes and methods for the detection of Listeria |
| 7645582 | Aptamers that bind to Listeria surface proteins |
| 20040072279 | Culture medium for detecting bacteria of Listeria genus |
| 7351548 | Culture medium for detecting bacteria of Listeria genus |
| 7439022 | Nucleic acids for detection of Listeria |
| 5932415 | Processes and agents for detecting Listerias |
| 5294537 | Monoclonal antibody assay for *Listeria monocytogenes* |
| 6951925 | Processes and agents for detecting Listerias |
| 5134063 | Methods for detection, identification and specification of Listerias |
| 5824468 | Detection of Listeria by means of recombinant bacteriophages |
| 5550022 | Method for the determination of pathogenic Listeria bacteria |
| | Nucleic acids which preferably hybridize under the hybridization conditions 5-6x SSC and 42° C. to 60° C. with a 2.5 kb large Kpnl-BamHl fragment from pLM63, preferably with a nucleic acid. |
| 2013011862 | Rapid process for detection of microorganism with magnetic particles |
| | Immunomagnetic particles in suspension as well as a blocking agent molecule for forming microorganism-magnetic particles complexes. For developing the signal, a reaction between complexes and substrates is carried out, using an enzyme acting as a marker. |
| 2009232852 | Use of outer membrane protein a (ompa) in treatment/prevention/diagnosis of infections caused by *Klebsiella pneumoniae* and other gram-negative bacteria |

### Limitations and difficulties

The analysis of environments presents some restrictions coming from the limitations of the associated sampling and testing methods. Some sampling methods commonly used nowadays in food industries are sponges, swabs, wipes and agar contact plates, among others. Their main limitations are their low recovery rates (lower than 10%, in some cases) and the restrictions as for the type of surfaces that can be sampled (limited size and, in some cases, only flat surfaces). With regard to current detection methods for routine monitoring of contamination in production plants, they comprise culture-, molecular- and immunoassay-based techniques, among others. The main limitations of these techniques result from the need of sample enrichment (because the limits of detection are not low enough) and of specialised equipment and personnel (which usually implies outsourcing the analyses).

These limitations, together with the cost of the analyses and the time to get the results, have a great influence on companies when establishing sampling frequencies and dispatching times. Nevertheless, they are allowed to use analytical methods other than the reference ones, in particular more rapid methods, as long as their use can provide equivalent results.

The last two US references listed above can be considered as the closest prior art. The patent application US2013011862 discloses a method for the rapid and sensitive determination of the presence of microorganism in a wide range of samples by means of immunomagnetic particles in suspension as well as a blocking agent molecule. Once the microorganism-magnetic particles complexes are obtained, a reaction between said complexes and substrates for developing the signal is carried out, using an enzyme acting as a marker. Contrary to the teaching of said disclosure, the present method does not need any blocking agent or any enzyme for producing an analytical signal and for being selective, rapid and robust; the device for carrying out the claimed method also shows relevant features different from the system of D1, as the use of one or more magnets at the bottom of the reaction chamber as well as at the bottom of the detection chamber.

The present invention is also faster and simplest than the method of US2009232852, said disclosure being based on the use of magnetic particles coated with specific OmpA antibody molecules. In spite of the similarities, it goes without saying that the nature of the samples analyzed in the latter are completely different from the present case (body fluids against production and processing environments of food samples), not being evident for any skilled in the art any resemblance between the disclosures.

The overall objective of this invention is to take advantage of this possibility and develop new processes and instruments allowing food business operators, as well as other industries, to improve the analysis and control of the production environments and the assessment of the cleaning processes, with a particular focus on spoilage and pathogenic microorganisms such as *Listeria monocytogenes.* In fact, unlike the closest prior art cited, the present method and device allow simultaneously identifying and detecting different microorganisms present in a sample.

### SUMMARY OF THE INVENTION

The invention relates to a method and a device as disclosed in claims 1 and 8, respectively, for identification and detection of microorganisms in a sample, more specifically pathogens, or said in other words, a microorganism that is pathogenic. The pathogen is any target (chosen, selected) microorganism that may affect the human health, and whose identification and quantification may be of particular interest, for instance because they spoil products such as foods, water, and cosmetics. The target microorganism (or microorganisms) can be selected from the group consisting of bacteria, fungi and yeasts. The method is designed for the detection of any pathogen of concern, such as *Listeria monocytogenes, Salmonella, Campylobacter, Staphylococcus,* or *Aspergillus,* among others. The sample may be a solid, liquid or semi-solid sample taken from a surface, or may be any sample taken from foods, liquids, cosmetics, water, even the environment. In the most suitable case, the sample is in liquid form. Also in the most suitable case, the sample is taken and recovered from a hard surface, in spite of the fact that said method for detection can be used for any other place in which the microorganisms grow.

The essential method for identification and detection of microorganisms in a sample comprises, as defined in claim 1:
- carrying out at least one immunoassay reaction on a sample previously taken, for isolating and separating at least one chosen, target analyte that is a microorganism susceptible of being contained therein, by incubating the sample with one or more reagents that are antibodies, each one being specific to a target microorganism or bacterial species, conjugated to a magnetic particle in such a way that the target microorganism couples to said specific magnetic antibody during the incubation step and is subsequently separated from the rest of the sample by magnetism;
- carrying out a (consecutive) second immunoassay reaction on the sample previously subjected to the first immunoassay reaction, for marking the microorganisms previously coupled to the specific antibody conjugated to the magnetic particle, before quantifying thereof, by incubating the sample with at least one second reagent, that is an antibody specific to the target microorganism or bacterial species and conjugated to a fluorescent marker; and
- detecting and quantifying the amount of the separated microorganism or microorganisms previously coupled to the antibodies by illuminating the same and measuring the emitted fluorescence.

Then, after the two immunoassay incubations, the separated and marked microorganism/s is/are then illuminated, and the amount of said microorganism is quantified by measuring the emitted fluorescence.

It is obviously derived from the description above that the method may be applied for detecting either only one type of microorganism or more than one type of microorganism at the same time. This way, in one embodiment it is possible to use more than one antibody at a time, each one of them for a specific microorganism, for carrying out the immunoassay reaction, with the aim of isolating and separating the different microorganisms from the rest of the sample. Each antibody must be a specific antibody to a particular target microorganism. In the present method, one of the antibodies is preferably an antibody specific to *Listeria monocytogenes,* and is named mAb2B3 (see examples).

The present invention also relates to a device as disclosed in claim 8 for identification and detection of microorganisms in a sample configured to carry out the method defined above, having a detection subsystem that comprises:
- a reaction unit, having
   ∘ a reaction chamber in the form of a deposit in which the taken sample is introduced by a first inlet and collected, and where the first and second immunoassay reactions for separating and marking the target microorganism take place by adding the specific antibodies as reagents by a second inlet, by means of a programmed automata; and
   ∘ one magnet (or more, preferably two) located at the bottom of the reaction chamber for retaining the target microorganism conjugated with the antibody comprising a magnetic particle by magnetism during the incubation reaction; connected to
- a detection unit, having
   ∘ a detection chamber to which the separated and marked microorganism is transported after the immunoassay reactions are complete, for quantifying the amount thereof; and
   ∘ one magnet located at the bottom of the detection chamber for retaining the separated and marked microorganism by magnetism during the quantification step;
- a source of light directed to the detection chamber, and
- at least one sensor (detector) for collecting and measuring the fluorescence emitted for the markers when excited into the detection chamber.

### DETAILED DESCRIPTION OF THE INVENTION

In a preferred embodiment, the magnetic particle is comprised of a magnetic core and a reactive layer that enables its suspension in water and its attachment to antibodies. Preferably, the magnetic particle is an iron oxide magnetic particle that is functionalized. The magnetic particles preferably have a size in the nanometer range (having more preferably a size lower or equal to 50 nm), which is a important feature for allowing the reaction in a suitable manner, and are preferably coated by a layer that enables its conjugation to the antibody, being more preferably a polymeric layer containing reactive groups (for example, carboxylic groups) that facilitate the bonds with other compounds (in this case, the specific antibody). The reagent usually used is the specific antibody already conjugated to the magnetic particle (although it is possible to add to the method a previous step for conjugating the antibody to the magnetic particle). For instance, the conjugation of one specific antibody to the nanomagnetic particle preferably occurs through reaction of a carboxylic group of the nanomagnetic particle polymeric coating with an amine group of the antibody. The antibody-magnetic particle conjugate can then be added to the reaction medium containing the microorganisms to allow binding of the antibodies to the bacterial cells.

In the more preferred case, the magnetic particles bound to the specific antibody are particles comprising an iron oxide core with dimensions in the range of nanometers, preferably 2 to 50 nm diameter. The metallic core is coated by an amphiphilic coating that allows suspension of the magnetic particles in water. This coating usually consists of a polymeric layer with carboxylic groups on its outer surface and a thickness of 2 to 5 nm. An example of this kind of magnetic particles can be found in Yang et al.: "Ultrasensitive detection and molecular imaging with magnetic nanoparticles", Analyst (2008), 133, 154-160. Conjugation of antibodies to these magnetic particles is achieved through reaction of a carboxylic group of the particle coating and an amine group of the antibody.

In one particular embodiment, the second specific antibody or antibodies are different from those used in the first immunoassay reaction. In another different embodiment, the second antibody specific to a target microorganism is the same as the one used for the separating immunoassay reaction, in such a way that said antibody is not linked to a magnetic particle but to a marker; this is a more preferred case for the present method. Therefore, in a more particular embodiment, the identification and detection method comprises:
- carrying out a first immunoassay reaction on a sample previously taken, for separating one or more target microorganisms by incubating said sample with at least one first specific antibody conjugated to a magnetic particle;
- carrying out a second immunoassay reaction on the sample, for marking the target microorganisms previously coupled to the first specific antibodies by incubating the sample with the same antibodies specific to the target microorganisms, but conjugated to a fluorescent marker (and not to magnetic particles); and
- detecting and quantifying the amount of the target microorganisms previously coupled to the antibodies by illuminating the same and measuring the emitted fluorescence.

Preferably, the antibody is linked to biotin for facilitating its conjugation with the fluorescent marker. For example, the antibody can be subjected to a previous biotinilation process, which consists in anchoring the antibody to the biotin.

It must be taken into account that, according to the description of the essential invention the second reagent that is a specific antibody is already conjugated to the fluorescent marker before the microorganism couples said antibody. However, said disclosure comprises two possibilities: In one particular embodiment, the second immunoassay reaction is carried out by adding one or more specific antibodies already conjugated to the fluorescent markers. In another particular embodiment, the second immunoassay reaction is carried out by adding simultaneously a reagent that is the antibody (or antibodies), and a reagent that is the fluorescent marker or markers, in such a way that during the immunoassay reaction the antibody firstly couples the fluorescent marker and secondly couples the microorganism. In this latter embodiment, the antibody is preferably linked to biotin.

The fluorescent markers are preferably quantum dots (such as Qdots®).

The detection is preferably performed through measurement of optical signal emitted after excitation of the sample, in such a way that the separated and marked microorganism is then illuminated with light of a specific wavelength for exciting the used fluorescent markers, and then the fluorescence emitted is collected and measured by a sensor. Basically, the method finally requires calculating the fluorescence intensity values and correlating to a concentration of microorganisms in the sample.

Preferably, the sample is washed after each immunoassay reaction, in such a way that the method then comprises the following steps:
- carrying out a first immunoassay reaction on a sample previously taken, for separating one or more target microorganisms by incubating and coupling said microorganisms to specific antibody or antibodies bound to magnetic particles;
- retaining said microorganisms coupled to the magnetic antibodies by means of magnetism, by activating one or more magnets;
- washing the sample for isolating and separating the antibody conjugated microorganisms;
- carrying out a second immunoassay reaction, for marking the microorganisms coupled to the magnetic antibodies by incubating and coupling thereof to a second specific antibody or antibodies, which may be preferably the same as the first antibodies, but conjugated to a fluorescent marker;
- retaining said marked microorganisms by means of magnetism, by activating one magnet;
- washing the sample for separating the isolated microorganisms conjugated to the antibodies; and
- detecting and quantifying the amount of the marked microorganisms by illuminating the same and measuring the emitted fluorescence.

Although the method described above can be applied for a sample previously taken not matter how and preferably presented in a liquid form, said method can also comprise a previous step of taking at least one sample, for recovering and collecting one or more types of microorganisms contained therein. Therefore, a complete preferred method comprises the following steps previous to the immunoassay reaction/s:
a) taking one or more samples (for recovering microorganisms);
b) separating/isolating by magnetism one or more target microorganisms of specific species by means of immunoassay techniques involving coupling of the microorganisms to antibodies conjugated with magnetic particles;
c) marking the target microorganisms by immunoassay techniques involving coupling of the separated microorganisms to antibodies conjugated with fluorescent markers; and
c) detecting and quantifying the amount of separated/isolated microorganisms.

Preferably, the sample is taken and recovered by injecting pressurized air with water in vacuum conditions for expelling the sample from its place of origin. Then, the expelled sample can be collected in a liquid as a liquid sample, before carrying out the immunoassay reactions. The sample can be selected and taken from a single sampling point, or can be taken from a sampling area defined by more than one sampling point.

With regard to the device object of the invention described above, the source of light for exciting the marked microorganism is preferably a LED of specific wavelength, said wavelength being selected depending on the fluorescent marker. Preferably, the sensor is selected from the group consisting of a CCD sensor, a silicon photomultiplier sensor, and both.

Besides, the sensor preferably comprises at least one filter, which the emitted light passes through once the marked microorganism is excited. The sensor can also contain a second filter, which the excitation light passes through and which blocks light of wavelengths different to that of excitation of the fluorescent marker. Said filters are optical filters and are selected in accordance to excitation and emission wavelengths. The filters can be preferably located in a cube, and can be then the following three filters: an excitation filter, a dichroid beam splitter that reflects excitation light and transmits emitted light from the sample, and an emission filter that allows only the emission wavelength to the detector.

Besides, in the most preferred case, the device can be coupled to a sampling subsystem connected to the detection subsystem (but that works independently), more specifically to the reaction chamber inlet, that acts by means of pressurized air and water in vacuum conditions and comprises:
- a sampling tip, that attaches to the place (for example a hard surface) from which the sample is taken, and contains
- a main cavity that defines the testing area and within which the pressurized air and water are applied for expelling and extracting the sample, said cavity having
- an inlet opening for injecting the pressurized air and water into the cavity, and
- an outlet opening for expelling and extracting the sample taken with the injected water.

When the identification and detection device comprises the two parts disclosed, the sampling and the detection subsystems, then the water containing the sample expelled by the former is transported and collected in the reaction chamber of the latter. Preferably, the sampling tip is assembled or attached to the place from which the sample is taken by means of vacuum. In this case, the vacuum conditions are achieved by means of a second cavity surrounding the outer surface of the testing area defined by the main cavity, said second cavity connected to additional means for creating vacuum therein.

The invention described consists of a method and an apparatus for carrying out said method, which comprises two different stages and subsystems, respectively: sampling and detection stages/subsystems. Both can perform independently or are preferably integrated to perform in a coordinated manner.

The most preferred detection subsystem is itself comprised of a reaction unit and a detector unit, according to the above, and is described next. The reaction unit contains a reactor chamber or deposit where the sample is collected and the immunoassay reaction takes place. It also contains the circuits for transportation of fluids from the sampling subsystem and to the detection unit. The reaction unit allocates one container for each different reagent used in the immunoassay and circuits for connecting these containers with the main reaction chamber. The reaction chamber presents an opening in its upper side that allows introduction of the sample from the sampling subsystem and the reagents from the reagents containers. The reaction chamber presents also a valve-regulated opening in its bottom side, connected to the detection unit. Transportation of fluids is regulated by a system of valves and peristaltic pumps controlled by a programmable automata. Additionally, a magnet is located at the bottom of the reaction chamber. Said magnet can be switched between two positions, one separated from the reaction chamber and one in contact with the walls of the reaction chamber. Magnet switching is performed by injection of pressurized air and can be controlled from the automata.

The preferred detection unit is based on the concept of fluorescence. The sample under measurement contains fluorescent quantum dots as fluorescent markers bound to pathogen specific antibodies. The sample is excited inside the detection chamber at a specific wavelength using the light source and the fluorescence emitted in turn is captured using the sensor/detector. Optical filters and a beam-splitter are mounted in a cube and placed to filter additional breakthrough and collect only the desired wavelength, whose intensity is measured.

Preferably, the detection unit comprises a measurement dark box that divides into two compartments. Each compartment encloses an excitation light source and a detector 1 and 2, respectively. An optical filter cube is switched between both the compartments. Both the compartments are independent of each other and are capable of functioning as standalone detection units. The use of two detectors is to provide verification and assurance of acquired results. The excitation light source is preferably a LED of specific wavelength. The wavelength of the source can be selected based on the fluorescent quantum dot used in the immunoassay during sample preparation. The optical filter cube contains optical filters that can be selected in accordance to excitation and emission wavelengths. Three different filters are housed in the cube: an excitation filter that filters only the incoming excitation wavelength, a dichroic beam-splitter that reflects excitation light and transmits emitted light from the sample, and finally an emission filter that allows only the emission wavelength to the detector.

This way, it is possible to detect any food-borne pathogen just by changing the filters with respect to the excitation and emission characteristics of the fluorescent markers (quantum dots) used in the immunoassay. The detector 1 is a SiPM detector module: Silicon photomultipliers are available in a wide range sensitive to different wavelengths and in different sizes. The detector module encloses a silicon photomultiplier that is sensitive to the emission wavelength with supporting electronics. A lens is used to adjust the focus. The user initiates measurement by pushing a button after which light collected from the sample is analysed and displayed on an LCD screen in the form of counts and a decision- PASS or FAIL indicating the absence or presence of the pathogen in the sample. Detailed analysis and data logging can be done by connecting to a computer. The detector 2 is a CCD camera: The second compartment of the detection unit has a CCD camera with a lens for focus that is software controlled. The user can enter a required integration time for which the sensor collects light from the sample and the captured image is displayed on screen. The intensity of the image is indicative of presence or absence of the pathogen under study.

The magnetic separation system, made out of an engineered thermoplastic is designed to be a part of a PLC flow system that transfers the collected sample to the detection subsystem in a semi-automated method. The assembly is placed inside the detection unit with inlet and outlet tubes connecting it to the flow system. The objective of the magnetic separation system is to separate and concentrate only the pathogen under study in the sample holder while washing out all the unbound fluorescent quantum dots. Separation is achieved by movement of a disc magnet towards and away the sample holder. The magnet is placed in an arm that is attached to a rotary solenoid. A small control PCB to drive the solenoid is also enclosed within the separation system and powered from the SiPM detector module. Pulsing the solenoid provides a 45 degree movement of the arm with the magnet thereby trapping only the pathogen bound to the magnetic particles for measurement. By using the ideal combination of immunoassay, optical filters and Silicon photomultiplier, the detection unit can be used for a speedy detection of any microorganism of interest.

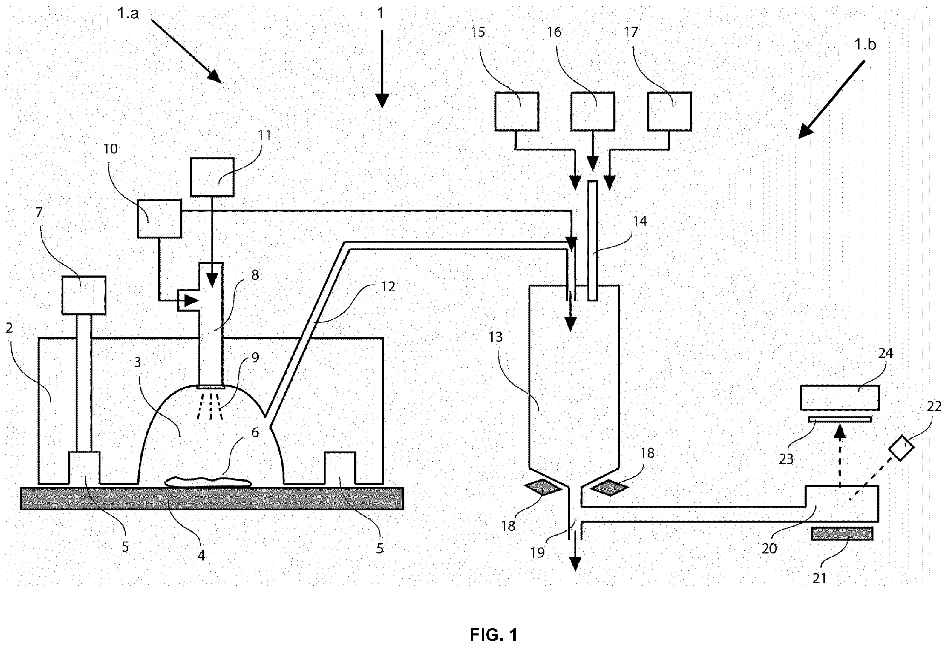

A preferred sampling subsystem that can be coupled to the detection subsystem of the instant invention according to the description above is shown in Figure 1 of this text (1.a). The sampling subsystem specifically consists of a sampling tip that houses a cavity whose shape defines the testing area on a surface. A circuit connects to at least one inlet opening into de main cavity and is configured to project (inject for the present device) at least one fluid into the area to be tested, such as water and air in the case of the present invention. A second circuit is connected to at least one outlet opening into the main cavity and is configured to retrieve a portion of at least one of the fluids projected (expelled) containing elements removed from the testing area. For creating vacuum conditions when taken the sample, the housing includes an additional cavity having an opening surrounding the outer surface of the treatment area, the sampling system further comprising an additional unit connected to the cavity and configured to create a vacuum within the additional cavity. Thanks to the vacuum made in the additional cavity, the device may be maintained adhered against the surface without the intervention of an external operator. Furthermore the device may be used for removing contaminants from a surface in slope, even when the inlet orifice of the main cavity is located below the surface. The sampling subsystem comprises a first hydraulic circuit for circulating a liquid connected to at least one inlet. Thus, by projecting a liquid into the main cavity, contaminants may be detached from the testing surface. A second circuit is connected to the outlet of the main cavity, which circulates a portion of the projected fluid and collects it into a recovery container. One example of this type of configuration for the sampling subsystem is disclosed in patent application FR14/01251.

It should be taken into account that in a preferred embodiment the device described above may comprise more than one reaction unit, thus allowing carrying out more than one first immunoassay reaction at a time, and thus analyzing more than one sample at the same time. In this preferred embodiment, several samples can be collected from the sampling subsystem and can be processed in different compartments containing each one of them a reaction chamber, the corresponding magnets and the circuitry for introduction of the sample and the reagents and an outlet for conduction of the sample to the detection system. The different samples can be transferred to the detection unit sequentially following completion of the immunoassay reactions in each reaction unit. Then, detection and quantification of microorganism levels is performed in the way previously described.

According to the present invention, the method for identification, collection and detection of microorganisms from surfaces can be carried out with the described device following these steps:
- selecting sampling points for defining a sampling area;
- covering the sampling area with the open cavity of the sampling tip;
- applying vacuum in the additional cavity to adhere the sampling tip to the surface;
- injecting a mixture of pressurized air and water into the main cavity of the sampling tip;
- collecting the liquid containing contaminants (the sample) into a reaction chamber that is a deposit;
- adding antibodies bound to magnetic particles to the reaction deposit and incubating the mixture;
- activating the magnet and subsequently washing off the reaction chamber;
- adding the same type of antibodies but this time bound to fluorescent markers, and incubating the mixture, while magnet is off;
- activating magnet and washing off reaction chamber;
- deactivating the magnet and washing off the antibodies-bound cells to the detector;
- activating the magnet at the detector for fixing the antibodies-bound cells;
- illuminating the marked microorganism inside the detection chamber with light of specific wavelength; and
- measuring the light intensity emitted.

The invention describes a method that enables highly efficient, fast and sensitive detection of specific microorganisms. This method is based on an immunoassay reaction that captures and isolated the microorganism to be tested, coupled to a detection method based on excitation of the sample and measurement of light emitted. Some of the performance features unique to this method are listed below:
- Antibodies can be selected for high selectivity, thus ensuring only the desired bacterial species or strain react and is detected.
- Different microorganisms can be identified simultaneously by using specific antibodies for said microorganisms.
- The immunoassay reaction usually takes 30 minutes to 2 hours to complete. Thus, reaction times are fast compared to incubation methods that require at least 24 hours.
- The detection method is fast, robust and highly sensitive as excitation and emission wavelengths are isolated using appropriate filters.

To add more, the invention describes an apparatus to perform sampling and detection of microorganism based on the method of the invention. There are performance features unique to this device, which are the following:
- The device may perform detecting and sampling operations independently, or all operations from sampling to detection, therefore negating in this latter case the need of specialized microbiology materials or facilities
- All operations can be programmed using actuators, therefore minimizing the need for personnel involvement.
- The cited sampling tip allows high rates of microorganism recovery from surfaces. Tests performed show that recovery rates are normally higher than 90 %, while swabs, plates and wipes commonly used for sampling surfaces usually recover between 4 and 10 % of the microorganisms on the surface.
- Cell viability is not affected by the recovery method using the sampling tip. This enables further incubation and growing of recovered cells if desired.

The invention provides a methodology and the required tools to perform just one or all the stages involved in diagnostics of the presence of unwanted microorganisms on places, such as surfaces, or on foods, drinks, the environment, etc. from sampling to isolation and measurement of levels of microbial contamination. In this sense, the invention substitutes traditional methodologies for control of microorganisms that require several stages to perform the same process. Potentially, any microorganism of concern can be detected with the present invention, such as *Listeria monocytogenes, Salmonella, Campylobacter, Staphylococcus, Aspergillus,* etc., by using the adequate antibodies to perform the immunoassay.

The invention is of application to control of the presence of microorganisms on places, preferably hard surfaces, which is especially relevant in industries such as food production, pharmaceutics, hospitals, environmental control, etc. Further, due to the ability of sampling and detection subsystems to operate independently, the invention can find application in enhanced sampling of surfaces for microbial control using traditional techniques, or to microbial analysis of other kinds of samples such as foods, liquids, cosmetics, water, environmental samples, etc. using only the detection subsystem.

The instrument developed is eventually aimed at providing user companies, such as food industries, with several advantages for detection of pathogens, especially on surfaces. Firstly, the easy interfacing of both the sampling and measuring stages, that are interfaced and automatized. This allows companies to carry out their microorganism contamination monitoring programs in a more quick, efficient and safe manner than with current tools. Nevertheless and despite that, the measuring subsystem have been developed independently from the sampling subsystem. This enables their separate use and would result in greater flexibility of the whole system and method. Moreover, the approach to the design and the operation of the system would be set up from a general perspective to ensure an easy application to the detection of other important pathogens as well as the detection of *Listeria monocytogenes* in other sectors, such as clinical and environmental.

The present invention represents a disruptive step in the development of biosensors for microbiological food safety analysis, since it has the potential to:
- Overcome the great limitation in current surface sampling methods due to their low recovery rates.
- Omit sample pre-enrichment steps thus leading to considerably shorter assay times.
- Achieve a higher sensitivity than current bioassays.

The innovative aspects of the invention are summarized in Table 2.

**Table 2. Summary of the innovative characteristics of the invention.**

| **Methods** | **Current situation** | **Present method/device** |
|---|---|---|
| Detection step | Need for sample pre-enrichment | No need for sample pre-enrichment |
| | Manual sample | Automated processes. |
| Sampling step | ▪ Low recovery rates (up to 10%) | High recovery rates (over 90%) |
| | ▪ Areas up to 1 m can be sampled. | No restriction regarding the outline and the size of the area to be sampled. |

### BRIEF DESCRIPTION OF THE FIGURES

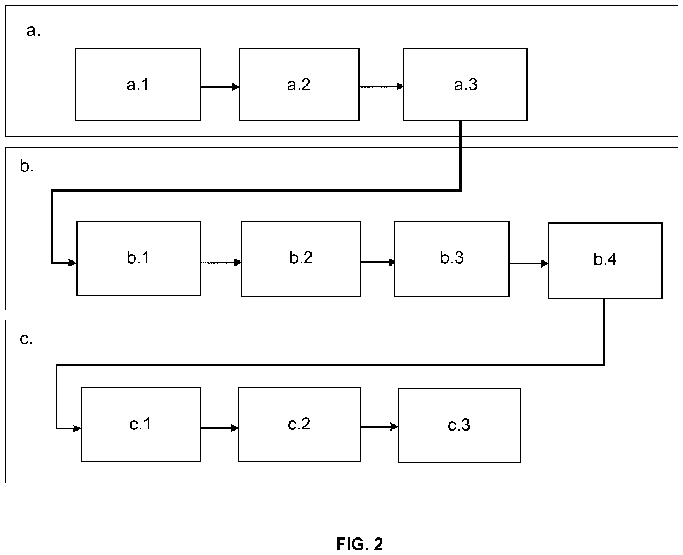

**FIG. 1.** Figure 1 shows a scheme of a preferred embodiment of the apparatus of invention having both, a sampling subsystem (1.a) and a detection subsystem (2), for the identification and detection of microorganism in a sample according to the method shown in Figure 2. The complete device **1** contains multiple elements to allow for sampling and detection of microorganisms on surfaces, and thus having a sampling subsystem (1.a) and a detection subsystem (1.b). For recovery of microorganisms, a sampling tip **2** that presents a main cavity **3** is placed on a hard surface **4.** Adherence of the sampling tip to the surface is improved by a concentric cavity **5** where vacuum is applied by means of a vacuum generator **7.** The main cavity **3** recovers microorganisms from a contaminated spot **6.** The sampling tip contains a tube **8** that projects a mixture of pressurized air and water **9** into the main cavity. Water and pressurized air are supplied by a water deposit **10** and an air compressor **11.** As **9** impacts on **6,** microorganisms are detached and leave the main cavity through a tube **12** that ends into the detection subsystem (1.b), more specifically into the reaction chamber **13.** A second inlet **14** injects reagents into the reaction chamber from deposits **15, 16** and **17,** containing preincubated antibodies conjugated to magnetic particles, antibodies preferably linked to biotin for facilitating the conjugation of the antibody to fluorescent markers, and fluorescent markers (such as quantum dots -Qdots®-), respectively. Two magnets **18** are placed at the bottom of the reaction chamber **13,** surrounding a tube **19** that directs fluid from the reaction chamber **13** to waste or to the detection chamber **20.** A magnet **21** for retention or microorganisms bound to magnetic antibodies is located below the detection chamber **20.** Detection of microorganisms is achieved by illumination of sample in the detection chamber **20** with a source of light **22.** Excited fluorescent antibodies in the reaction chamber **20** emit light which passes through a filter **23** and is collected and measured by a detector **24.**
**FIG. 2.** Figure 2 shows the method for identification and detection of microorganisms in a (hard) surface described in this invention in a preferred embodiment, as a flow diagram. This scheme shows a method comprising the three main phases involved in control of microorganisms: a) recovery of a sample, b) immunoassay reaction and c) detection, as well as the individual steps involved in these three phases. The immunoassay reaction results in attachment of specific antibodies to the microorganism of interest.
   a) Taking samples (recovery or microorganism from surfaces):
      a.1) vacuum attachment of sampling tip to surface;
      a.2) injection or mixed pressurized air and water; and
      a.3) collection of a sample containing microorganisms;
   b) Immunoassay reactions:
      b.1) addition of antibodies bound to magnetic particles;
      b.2) magnet on and wash;
      b.3) incubate with antibodies bound to fluorescent markers; and
      b.4) magnet on and wash;
   c) Detection of microorganisms
      c.1) cell transport to detection chamber;
      c.2) illumination of cells; and
      c.3) quantification of fluorescence intensity.

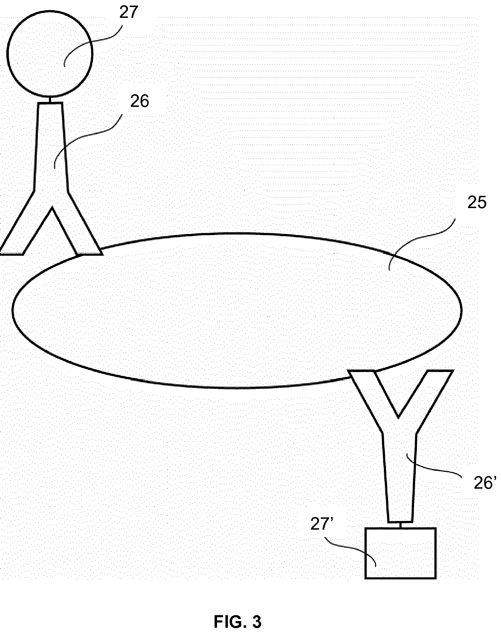

**FIG. 3.** Figure 3 shows a detailed scheme of the antibodies carrying different conjugates and coupled to a target microorganism recovered from a sample. Here, a bacteria cell **25** is coupled to a first specific antibody **26** carrying a conjugate that is a magnetic particle **27,** and to a second specific antibody **26',** which may be preferably the same as 26, carrying a conjugate that is a fluorescent marker **27'.**

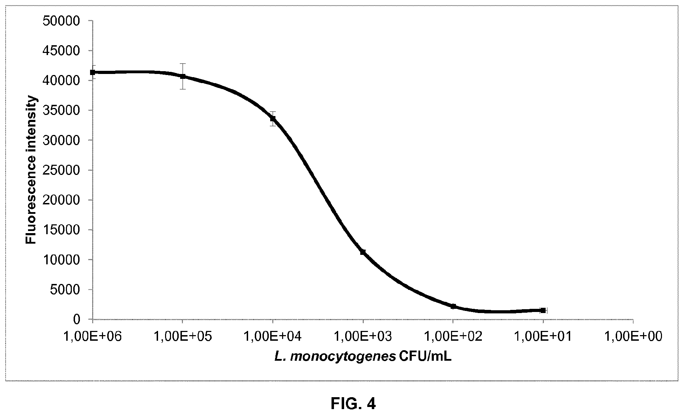

**FIG. 4****.** Measure of fluorescence intensity at 655 nm after excitation of samples containing different concentrations of *L. monocytogenes* bound to fluorescence and magnetic antibodies with light of 405 nm. according to Example 2.

### EXAMPLES

### Example 1. Determination of recovery rates of microorganisms in a sample taken for the purpose of the instant method and device.

*L. monocytogenes* biofilms were grown on stainless steel coupons according to standard procedures. The contaminated materials were sampled by means of the sampling subsystem of the invention and of conventional techniques (swabs and RODAC plates). Sampling with the sampling subsystem involved the following steps:
1. Compressed air + water during 1 min at 3 bar and 50 Lpm (Litres per minute).
2. Compressed air during 1 min at 3 bar and 50 Lpm.

The results of the sampling trials with the invention and with the conventional techniques are shown in Table 3 and Table 4:

**Table 3. Results of sampling L. monocytogenes biofilms grown on stainless coupons with the invention.**

| **L. monocytogenes (log CFU/surface)** | **Microorganisms recovered (log CFU) n=3** | **Detachment rate** |
|---|---|---|
| 5.58 | 4.58 | **82.08 %** |
| 5.64 | 4.69 | **83.16 %** |
| 5.64 | 5.06 | **89.71 %** |
| 5.35 | 4.56 | **85.23 %** |
| 5.00 | 4.58 | **91.60 %** |
| **5.00** | **4.73** | **94.6 %** |

**Table 4. Results of sampling L. monocytogenes biofilms grown on stainless steel coupons with conventional techniques.**

| **Theoretical inoculum (log CFU/sample)** | **Swab (log CFU/sample)** | **Rodac plate (log CFU/sample)** |
|---|---|---|
| 4.67 | 3.48 | **>2.18** |
| 5.43 | 2.95 | **>2.18** |
| **6.05** | **3.88** | **>2.18** |

Sampling with the invention yielded recovery rates between 82 and 95 %. The recovery rates using the conventional techniques were below 4%. More precisely, they were 3.43% using swabs and 1.26% using Rodac plates. This was due to the fact that the quantification limit of the latter was 2.18 log CFU/sample (150 CFU/sample). Although this represented a particular limitation of the Rodac plates, the main conclusion was that sampling with conventional techniques could lead to underestimate the actual levels of contamination in industries.

### Example 2. Immunoassay validation of method and device according to the instant invention.

Serial dilutions of *L. monocytogenes* were prepared in peptone saline solution and plated in selective media to calculate the control recovery rates. Afterwards, working solutions in the range 1E7 - 1 E4 CFU/mL were prepared in sterile water.

The detection protocol with the immunosensor involved the following steps:
1. Incubate mAb2B3-nanomagnetic particle conjugate (0.5 µg/mL, 100uL) coated beads with *L. monocytogenes* sample (1 mL) at 21 °C for 10 mins.
2. Inject into fluidic cell using flow-rate 1 mL/min ensuring the magnet is in place.
3. Wash the sample with 10 mL of distilled H₂0 at a flow-rate of 5 mL/min.
4. Inject into fluidic cell mAb2B3 (200 ng/mL, 1mL) at flow rate 1 mL/min with magnet in place.
5. Incubate at 21 °C for 10 min. Wash out unbound antibody with 10 mL of distilled H₂0 at a flow-rate of 5 mL/min with magnet in place.
6. Inject into fluidic cell streptavidin labelled Qdot® (0.02 µM, 1 mL) at flow rate 1 mL/min with magnet in place.
7. Incubate at 21 °C for 10 min. Wash out unbound antibody with 10 mL of distilled H₂0 at a flow-rate of 5 mL/min with magnet in place.
8. Measure fluorescence.

The steps described above where automatically executed in sequence using the device of the invention, and the whole process took around 40 minutes. Figure 4 shows the values of fluorescence obtained after excitation of samples containing different concentrations of *L. monocytogenes.* Signal intensity increases with increasing cell concentration, and a difference against baseline signal can be detected from values of 100 CFU/mL.

### Example 3. Detection of marked cells

Samples containing different concentrations of *L. monocytogenes* cells bound to magnetic and fluorescent antibodies where placed in the detection unit of the device described by this invention. Table 5 shows values expressed by the detector in its silicon photomultiplier version. Volume of detection chamber is 1 mL, therefore samples content can be expressed as number of cells. Results obtained show that the detector can provide a positive value from a content of 10 cells, thus confirming the high sensitivity of this method.

**Table 5. Values expressed by the silicon photomultiplier version of the detection subsystem after excitation of samples containing different concentrations of L. monocytogenes bound to fluorescence and magnetic antibodies with light of 405 nm.**

| **SAMPLE** | **SIGNAL** | **NOISE** | **RESULT** |
|---|---|---|---|
| EMPTY CHAMBER | 636 | 598 | 41 |
| TAP WATER | 642 | 598 | 43 |
| ISOPROPANOL | 669 | 597 | 71 |
| 10 cells | 717 | 601 | 71 |
| 10e2 cells | 811 | 598 | 213 |
| 10e3 cells | 1633 | 605 | 1027 |
| 10e4 cells | 14822 | 594 | 14227 |
| 10e5 cells | 15223 | 602 | 14620 |

## Claims

1. A method for identification and detection of microorganisms in a sample, **characterized in that** it comprises:
- carrying out at least one immunoassay reaction on a sample previously taken, for isolating and separating at least one chosen, target analyte that is a microorganism susceptible of being contained therein, by incubating the sample with one or more reagents that are antibodies, each one being specific to a target microorganism, conjugated to a magnetic particle, in such a way that the target microorganism couples to the specific magnetic antibody during the incubation and is subsequently separated from the rest of the sample by magnetism;
- carrying out a second immunoassay reaction on the sample previously subjected to the first immunoassay reaction, for marking the microorganism previously coupled to the specific antibody conjugated to the magnetic particle before quantifying thereof, by incubating the sample with at least one second reagent, that is an antibody specific to the target microorganism conjugated to a fluorescent marker; and
- detecting and quantifying the amount of the separated microorganism previously coupled to the antibodies by illuminating the same and measuring the emitted fluorescence.

2. The method according to claim 1, wherein the magnetic particle has a size in the nanometer range, and comprises a magnetic core coated with a reactive layer that enables its suspension in water and its conjugation to the antibody.

3. The method according to claim 2, wherein the magnetic particle is an iron oxide magnetic particle.

4. The method according to any one of previous claims 1 to 3, wherein the at least one second antibody is different from the antibody used in the first immunoassay reaction, or the at least one second antibody is the same as the antibody used in the first immunoassay reaction, but linked to a fluorescent marker.

5. The method according to any one of previous claims, wherein it comprises the following steps:
- carrying out a first immunoassay reaction on a sample previously taken, for separating at least one target microorganism by incubating and coupling said microorganism to at least one specific antibody bound to a magnetic particle;
- retaining said microorganisms coupled to the magnetic antibodies by means of magnetism, by activating one or more magnets;
- washing the sample for isolating and separating the microorganisms conjugated to the antibodies;
- carrying out a second immunoassay reaction, for marking the microorganisms coupled to the magnetic antibodies by incubating and coupling thereof to at least one second specific antibody that is of the same type that those used in the first immunoassay reaction but conjugated to a fluorescent marker;
- retaining said marked microorganisms by means of magnetism, by activating one magnet;
- washing the sample for separating the isolated microorganisms conjugated to the antibodies; and
- detecting and quantifying the amount of the marked microorganisms by illuminating the same and measuring the emitted fluorescence.

6. The method according to any one of the previous claims, wherein the immunoassay reactions are carried out by using more than one antibody, each one of them being specific of a particular microorganism, thus enabling simultaneous detection of different microorganisms.

7. The method according to any one of the previous claims, wherein the sample is previously taken and recovered by injecting pressurized air with water in vacuum conditions for expelling the sample from its place of origin and collecting it in a liquid for the immunoassay reaction.

8. A device for identification and detection of microorganisms in a sample configured to carry out the method described in any one of the previous claims, **characterized in that** it has a detection subsystem that comprises:
- a reaction unit, having
∘ a reaction chamber in the form of a deposit in which the taken sample is introduced by a first inlet and collected, and where the first and second immunoassay reactions for separating and marking the target microorganism take place by adding the specific antibodies as reagents by a second inlet, by means of a programmed automata; and
∘ one or more magnets located at the bottom of the reaction chamber for retaining the target microorganism conjugated with the antibody comprising a magnetic particle by magnetism during the incubation reaction; connected to
- a detection unit, having
∘ a detection chamber to which the separated and marked microorganism is transported after the immunoassay reactions are complete, for quantifying the amount thereof; and
∘ one magnet located at the bottom of the detection chamber for retaining the separated and marked microorganism by magnetism during the quantification step;
- a source of light directed to the detection chamber, and
- at least one sensor for collecting and measuring the fluorescence emitted for the markers when excited into the detection chamber.

9. The device according to claim 8, wherein the source of light for exciting the marked microorganism is a LED of a wavelength corresponding to the fluorescent marker, and the sensor is selected from the group consisting of a CCD sensor, a silicon photomultiplier sensor, and both.

10. The device according to any one of claims 8 or 9, wherein the sensor comprises at least one optical filter, for isolation of the excitation and emission wavelengths.

11. The device according to claim 10, wherein it comprises three filters located in a cube with a beam-splitter, and are: an excitation filter; a dichroid beam splitter that reflects excitation light and transmits emitted light from the sample; and an emission filter that allows only the emission wavelength to the sensor.

12. The device according to any one of claims 8 to 11, wherein it comprises a sampling subsystem coupled and connected to the reaction chamber of the detection subsystem, that acts by means of pressurized air and water and comprises:
- a sampling tip, that attaches to the place from which the sample is taken, and contains
- a main cavity that defines the testing area and within which the pressurized air and water are applied for expelling and extracting the sample; said cavity having
- an inlet opening for injecting the pressurized air and water into the cavity;
- an outlet opening for expelling and extracting the sample taken with the injected water; and
- a second cavity surrounding the outer surface of the testing area and which applies vacuum to adhere the sampling tip to the surface.

13. The device according to any one of claims 8 to 12, wherein the reaction unit contains:
- circuits for transportation of fluids from the sampling subsystem and to the detection unit;
- one container for each different reagent used in the immunoassay reaction; and
- circuits for connecting these containers with the reaction chamber;
- and the reaction chamber presenting:
- an opening in its upper side that allows introduction of the sample from the sampling subsystem and the reagents from the reagents containers;
- a valve-regulated opening in its bottom side, connected to the detection unit;
and wherein transportation of fluids is regulated by a system of valves and peristaltic pumps controlled by a programmable automata.

14. The device according to any one of claims 8 to 13, wherein it comprises more than one reaction unit for carrying out more than one first immunoassay reaction at the same time, and analyzing more than one sample at the same time.

## Patentansprüche

1. Verfahren zur Identifizierung und Erfassung von Mikroorganismen in einer Probe, **dadurch gekennzeichnet, dass** das Verfahren umfasst:
- Ausführen mindestens einer Immuntestreaktion an einer Probe, die zuvor genommen wurde, um mindestens einen ausgewählten Zielanalyten, der ein Mikroorganismus ist und beeinflussbar ist, darin enthalten zu sein, zu isolieren und zu separieren durch Bebrüten der Probe mit einem oder mehreren Reagenzien, die Antikörper sind, wovon jeder speziell für einen Zielmikroorganismus ist, der bzw. die mit einem magnetischen Teilchen so gepaart ist bzw. sind, dass der Zielmikroorganismus an den speziellen magnetischen Antikörper während des Bebrütens ankoppelt und nachfolgend von dem Rest der Probe magnetisch separiert wird;
- Ausführen einer zweiten Immuntestreaktion an der Probe, die zuvor der ersten Immuntestreaktion unterzogen wurde, um den zuvor an den speziellen Antikörper angekoppelte Mikroorganismus, der mit dem magnetischen Teilchen gepaart ist, vor seiner Quantifizierung zu markieren, indem die Probe mit mindestens einem zweiten Reagenz bebrütet wird, das ein Antikörper ist, der speziell für den Zielmikroorganismus ist, der mit einem fluoreszierenden Markierungsstoff gepaart ist; und
- Erfassen und Quantifizieren der Menge des separierten Mikroorganismus, der zuvor an die Antikörper gekoppelt war, durch Beleuchten des Mikroorganismus und Messen der emittierten Fluoreszenz.

2. Verfahren nach Anspruch 1, wobei das magnetische Teilchen eine Größe im Nanometerbereich hat und einen magnetischen Kern aufweist, der mit einer reaktiven Schicht bedeckt ist, die seine Auflösung in Wasser und seine Paarung mit dem Antikörper ermöglicht.

3. Verfahren nach Anspruch 2, wobei das magnetische Teilchen ein magnetisches Eisenoxidteilchen ist.

4. Verfahren nach einem der vorhergehenden Ansprüche 1 bis 3, wobei der mindestens eine zweite Antikörper sich von dem in der ersten Immuntestreaktion verwendeten Antikörper unterscheidet, oder der mindestens eine zweite Antikörper der gleiche ist wie der in der ersten Immuntestreaktion verwendete Antikörper, aber mit einem fluoreszierenden Markierungsstoff verbunden ist.

5. Verfahren nach einem der vorhergehenden Ansprüche, wobei das Verfahren die folgenden Schritte umfasst:
- Ausführen einer ersten Immuntestreaktion an einer zuvor genommenen Probe zum Separieren mindestens eines Zielmikroorganismus durch Bebrüten und Ankoppeln des Mikroorganismus an den mindestens einen speziellen Antikörper, der an ein magnetisches Teilchen gebunden ist;
- Zurückhalten der Mikroorganismen, die mittels Magnetismus mit den magnetischen Antikörpern verbunden sind, durch Aktivieren eines oder mehrerer Magnete;
- Spülen der Probe zur Isolierung und Separierung der mit den Antikörpern gepaarten Mikroorganismen;
- Ausführen einer zweiten Immuntestreaktion, um die an die magnetischen Antikörper angekoppelten Mikroorganismen zu markieren, indem sie bebrütet und mit mindestens einem zweiten speziellen Antikörper gekoppelt werden, der von der gleichen Art ist wie jener, der in der ersten Immuntestreaktion verwendet wird, aber mit einem fluoreszierenden Markierungsstoff gepaart ist;
- Zurückhalten der markierten Mikroorganismen mittels Magnetismus durch Aktivieren eines Magneten;
- Spülen der Probe zum Trennen der isolierten Mikroorganismen, die mit den Antikörpern gepaart sind; und
- Erfassen und Quantifizieren der Menge der markierten Mikroorganismen durch Beleuchten der Mikroorganismen und durch Messen der emittierten Fluoreszenz.

6. Verfahren nach einem der vorhergehenden Ansprüche, wobei die Immuntestreaktionen ausgeführt werden unter Verwendung von mehr als einem Antikörper, wovon jeder spezifisch für einen speziellen Mikroorganismus ist, um damit eine gleichzeitige Erfassung unterschiedlicher Mikroorganismen zu ermöglichen.

7. Verfahren nach einem der vorhergehenden Ansprüche, wobei die Probe zuvor genommen und durch Injektion von unter Druckluft und Wasser unter Vakuumbedingungen wiederhergestellt wird, um die Probe von ihrem Ursprungsort auszutreiben und sie in einer Flüssigkeit für die Immuntestreaktion anzureichern.

8. Einrichtung zur Identifizierung und Erfassung von Mikroorganismen in einer Probe, wobei die Einrichtung ausgebildet ist, das in einem der vorhergehenden Ansprüche beschriebene Verfahren auszuführen, **dadurch gekennzeichnet, dass** die Einrichtung ein Erfassungssubsystem hat, das umfasst:
- eine Reaktionseinheit, die aufweist
o eine Reaktionskammer in Form einer Schicht, in die die genommene Probe durch einen ersten Einlass eingeführt und gesammelt wird, und wobei die erste und die zweite Immuntestreaktion zum Separieren und Markieren des Zielmikroorganismus stattfinden, indem die speziellen Antikörper als Reagenzien durch einen zweiten Einlass mittels einer programmierten Automaten hinzugefügt werden; und
o einen oder mehrere Magneten, die an dem Boden der Reaktionskammer zum Zurückhalten des Zielmikroorganismus mittels Magnetismus während der Bebrütungsreaktion, der mit dem ein magnetisches Teilchen enthaltenden Antikörper gepaart ist; verbunden mit
- einer Detektiereinheit, die aufweist
∘ eine Detektierkammer, in die der separierte und markierte Mikroorganismus nach Abschluss der Immuntestreaktionen zur Quantifizierung seiner Menge transportiert wird; und
∘ einen Magneten, der an dem Boden der Detektierkammer angeordnet ist, um den separierten und markierten Mikroorganismus durch Magnetismus während des Quantifizierungsschritts zurückzuhalten;
- eine Lichtquelle, die auf die Detektierkammer gerichtet ist, und
- mindestens einen Sensor zum Sammeln und Messen der für die Markierungsstoffe emittierten Fluoreszenz bei Anregung in der Detektierkammer.

9. Einrichtung nach Anspruch 8, wobei die Lichtquelle zum Anregen des markierten Mikroorganismus eine LED mit einer Wellenlänge ist, die dem fluoreszierenden Markierungsstoff entspricht, und der Sensor aus der Gruppe ausgewählt ist bestehend aus: einem CCD-Sensor, einem Silizium-Fotovervielfacher-Sensor oder beiden.

10. Einrichtung nach Anspruch 8 oder 9, wobei der Sensor mindestens einen optischen Filter zur Trennung der Anregungs- und Emissionswellenlänge aufweist.

11. Einrichtung nach Anspruch 10, die drei Filter aufweist, die in einem Würfel mit einem Strahlteiler angeordnet sind, und die vorgesehen sind in Form von: einem Anregungsfilter; einem dichroiden Strahlteiler, der Anregungslicht reflektiert und von der Probe emittiertes Licht durchlässt; und einem Emissionsfilter, der nur die Emissionswellenlänge zu dem Sensor durchlässt.

12. Einrichtung nach einem der Ansprüche 8 bis 11, die ein Probennahmesubsystem aufweist, das mit der Reaktionskammer des Detektiersubsystems gekoppelt und verbunden ist, das mittels Druckluft und Wasser arbeitet und aufweist:
- eine Abtastspitze, die an der Stelle haftet, von der die Probe genommen ist, und die enthält
- eine Hauptkavität, die den Prüfbereich abgrenzt und innerhalb derer die Druckluft und das Wasser zum Austreiben und Extrahieren der Probe angewendet werden; wobei die Kavität aufweist
- eine Einlassöffnung zum Einführen der Druckluft und des Wassers in die Kavität;
- eine Auslassöffnung zum Austreiben und Extrahieren der mit dem eingeführten Wasser genommenen Probe; und
- eine zweite Kavität, die die Außenfläche des Prüfbereichs umschließt und Vakuum ausübt, um die Abtastspitze an der Oberfläche anzuhaften.

13. Einrichtung nach einem der Ansprüche 8 bis 12, wobei die Reaktionseinheit enthält:
- Leitungen zum Transport von Fluiden aus dem Probennahmesubsystem zu der Detektiereinheit;
- einen Behälter für jedes unterschiedliche Reagenz, das in der Immuntestreaktion verwendet wird; und
- Leitungen zum Verbinden dieser Behälter mit der Reaktionskammer;
- und wobei die Reaktionskammer aufweist:
- eine Öffnung in ihrer oberen Seite, die das Einführen der Probe von dem Probennahmesubsystem und des Reagenz aus den Reagenzienbehältern ermöglicht;
- eine ventilgesteuerte Öffnung in ihrer Unterseite, die mit der Detektiereinheit verbunden ist;
und wobei der Transport von Fluiden durch ein System aus Ventilen und Peristaltikpumpen gesteuert ist, das durch einen programmierbaren Automaten gesteuert ist.

14. Einrichtung nach einem der Ansprüche 8 bis 13, wobei sie mehr als eine Reaktionseinheit zur gleichzeitigen Ausführung von mehr als einer ersten Immuntestreaktion und zur gleichzeitigen Analyse von mehr als einer Probe aufweist.

## Revendications

1. Procédé pour l'identification et la détection de microorganismes dans un échantillon, **caractérisé en ce qu'**il comprend les étapes suivantes :
réaliser au moins une réaction d'immunoessai sur un échantillon préalablement prélevé, pour isoler et séparer au moins un analyte cible choisi qui est un microorganisme susceptible d'y être contenu, en incubant l'échantillon avec un ou plusieurs réactifs qui sont des anticorps, chacun étant spécifique à un microorganisme cible, conjugué à une particule magnétique, de sorte que le microorganisme cible se couple à l'anticorps magnétique spécifique pendant l'incubation et est ensuite séparé du reste de l'échantillon par magnétisme ;
réaliser une seconde réaction d'immunoessai sur l'échantillon préalablement soumis à la première réaction d'immunoessai, pour marquer le microorganisme préalablement couplé à l'anticorps spécifique conjugué à la particule magnétique avant sa quantification, en incubant l'échantillon avec au moins un second réactif, qui est un anticorps spécifique au microorganisme cible conjugué à un marqueur fluorescent ; et
détecter et quantifier la quantité du microorganisme préalablement couplé aux anticorps en éclairant ce dernier et en mesurant la fluorescence émise.

2. Procédé selon la revendication 1, dans lequel la particule magnétique a une taille dans la plage nanométrique, et comprend un noyau magnétique recouvert avec une couche réactive qui permet sa suspension dans l'eau et sa conjugaison à l'anticorps.

3. Procédé selon la revendication 2, dans lequel la particule magnétique est une particule magnétique d'oxyde de fer.

4. Procédé selon l'une quelconque des revendications 1 à 3, dans lequel le au moins un second anticorps est différent de l'anticorps utilisé dans la première réaction d'immunoessai, ou bien le au moins un second anticorps est le même que l'anticorps utilisé dans la première réaction d'immunoessai, mais relié à un marqueur fluorescent.

5. Procédé selon l'une quelconque des revendications précédentes, dans lequel il comprend les étapes suivantes :
réaliser une première réaction d'immunoessai sur un échantillon préalablement prélevé, pour séparer au moins un microorganisme cible en incubant et en couplant ledit microorganisme à au moins un anticorps spécifique relié à une particule magnétique ;
retenir lesdits microorganismes couplés aux anticorps magnétiques au moyen du magnétisme, en activant un ou plusieurs aimants ;
laver l'échantillon pour isoler et séparer les microorganismes conjugués aux anticorps ;
réaliser une seconde réaction d'immunoessai, pour marquer les microorganismes couplés aux anticorps magnétiques par incubation et en les couplant au moins à un second anticorps spécifique qui est du même type que ceux utilisés dans la première réaction d'immunoessai mais conjugués à un marqueur fluorescent ;
retenir lesdits microorganismes marqués au moyen du magnétisme, en activant un aimant ;
laver l'échantillon pour séparer les microorganismes isolés conjugués aux anticorps ; et
détecter et quantifier la quantité des microorganismes marqués en éclairant ces derniers et en mesurant la fluorescence émise.

6. Procédé selon l'une quelconque des revendications précédentes, dans lequel les réactions d'immunoessai sont réalisées en utilisant plus d'un anticorps, chacun d'entre eux étant spécifique d'un microorganisme particulier, permettant ainsi la détection simultanée de différents microorganismes.

7. Procédé selon l'une quelconque des revendications précédentes, dans lequel l'échantillon est préalablement prélevé et recouvert en injectant de l'air sous pression avec de l'eau dans des conditions de vide pour expulser l'échantillon de sa place d'origine et le collecter dans un liquide pour la réaction d'immunoessai.

8. Dispositif pour l'identification et la détection de microorganismes dans un échantillon configuré pour réaliser le procédé décrit dans l'une quelconque des revendications précédentes, **caractérisé en ce qu'**il a un sous-système de détection qui comprend :
une unité de réaction ayant :
une chambre de réaction se présentant sous la forme d'un dépôt dans lequel l'échantillon prélevé est introduit par une première entrée et collecté, et où les première et seconde réactions d'immunoessai pour séparer et marquer le microorganisme cible ont lieu en ajoutant les anticorps spécifiques en tant que réactifs par une seconde entrée, au moyen d'un automate programmé ; et
un ou plusieurs aimants situés au fond de la chambre de réaction pour retenir le microorganisme cible conjugué avec l'anticorps comprenant une particule magnétique par magnétisme pendant la réaction d'incubation ; raccordée à :
une unité de détection ayant :
une chambre de détection vers laquelle le microorganisme séparé et marqué est transporté après l'achèvement des réactions d'immunoessai, pour quantifier leur quantité ; et
un aimant situé au fond de la chambre de détection pour retenir le microorganisme séparé et marqué par magnétisme pendant l'étape de quantification ;
une source de lumière dirigée vers la chambre de détection, et
au moins un capteur pour collecter et mesurer la fluorescence émise pour les marqueurs lorsqu'ils sont excités dans la chambre de détection.

9. Dispositif selon la revendication 8, dans lequel la source de lumière pour exciter le microorganisme marqué est une diode électroluminescente d'une longueur d'onde correspondant au marqueur fluorescent, et le capteur est sélectionné dans le groupe comprenant un capteur CCD, un capteur photomultiplicateur à silicium, et les deux.

10. Dispositif selon l'une quelconque des revendications 8 ou 9, dans lequel le capteur comprend au moins un filtre optique, pour l'isolation des longueurs d'onde d'excitation et d'émission.

11. Dispositif selon la revendication 10, dans lequel il comprend trois filtres positionnés dans un cube avec un séparateur de faisceau, et sont : un filtre d'excitation ; un séparateur de faisceau dichroïque qui reflète la lumière d'excitation et transmet la lumière émise à partir de l'échantillon ; et un filtre d'émission qui permet uniquement la longueur d'onde d'émission jusqu'au capteur.

12. Dispositif selon l'une quelconque des revendications 8 à 11, dans lequel il comprend un sous-système d'échantillonnage couplé et raccordé à la chambre de réaction du sous-système de détection, qui agit au moyen d'air sous pression et d'eau et comprend :
une pointe d'échantillonnage, qui se fixe à l'endroit où l'échantillon a été prélevé, et continent :
une cavité principale qui définit la zone de test et à l'intérieur de laquelle l'air sous pression et l'eau sont appliqués pour expulser et extraire l'échantillon ; ladite cavité rayant :
une ouverture d'entrée pour injecter l'air sous pression et l'eau dans la cavité ;
une ouverture de sortie pour expulser et extraire l'échantillon prélevé avec l'eau injectée ; et
une seconde cavité entourant la surface externe de la zone de test et qui applique du vide pour faire adhérer la pointe d'échantillonnage à la surface.

13. Dispositif selon l'une quelconque des revendications 8 à 12, dans lequel l'unité de réaction continent :
des circuits pour le transport des fluides du sous-système d'échantillonnage et vers l'unité de détection ;
un récipient pour chaque réactif différent utilisé dans la réaction d'immunoessai ; et
des circuits pour raccorder ces récipients avec la chambre de réaction ;
et la chambre de réaction présentant :
une ouverture dans son côté supérieur qui permet l'introduction de l'échantillon à partir du sous-système d'échantillonnage et des réactifs à partir des récipients de réactifs ;
une ouverture régulée par valve dans son côté inférieur, raccordée à l'unité de détection ;
et dans lequel le transport des fluides est régulé par un système de valves et de pompes péristaltiques contrôlées par un automate programmable.

14. Dispositif selon l'une quelconque des revendications 8 à 13, dans lequel il comprend plus d'une unité de réaction pour réaliser plus d'une première réaction d'immunoessai à la fois, et analyser plus d'un échantillon à la fois.
